# EUROPEAN PATENT SPECIFICATION

(11) **EP 3 226 831 B1**
(45) Date of publication and mention of the grant of the patent: **13.10.2021**
(21) Application number: 15807620.8
(22) Date of filing: 02.12.2015
(51) Int. Cl.: A61K 8/34, A61Q 1/02, A61K 8/87, A61K 8/06

(54) **COMPOSITIONS COMPRISING AN AUTOASSOCIATIVE POLYURETHANE, A FATTY ALCOHOL OR ACID, A NONIONIC SURFACTANT, AND A PIGMENT**
ZUSAMMENSETZUNGEN MIT EINEM AUTOASSOZIATIVEN POLYURETHAN, EINEM FETTALKOHOL ODER EINER SÄURE, EINEM NICHTIONISCHEN TENSID UND EINEM PIGMENT
COMPOSITIONS COMPRENANT UN POLYURÉTHANE AUTO-ASSOCIATIF, UN ACIDE OU ALCOOL GRAS, UN TENSIOACTIF NON-IONIQUE, ET UN PIGMENT

(30) Priority: 03.12.2014 FR 1461890
(43) Date of publication of application: 11.10.2017
(73) Proprietor: L'Oréal, 75008 Paris (FR)
(72) Inventor: BRUN, Gaëlle, 94152 CHEVILLY LA RUE (FR); COLLETTE, Annick, 94100 St Maur Des Fosses (FR)
(74) Representative: Lavoix
(86) International application number: PCT/EP2015/078430
(87) International publication number: WO 2016/087554

(56) References cited:
- EP-A1- 0 970 692
- EP-A1- 2 565 233
- EP-A2- 1 341 504
- FR-A1- 2 985 174
- None

## Description

The present invention concerns compositions, in particular cosmetic, comprising at least one autoassociative polyurethane, at least fatty alcohol or acid, at least one nonionic surfactant and at least one pigment, in the form of oil-in-water emulsions. These compositions are particularly intended for skin care and/or makeup.

Most foundations are water-in-oil emulsions resulting in a lack of freshness on application.

Foundations which are in oil-in-water emulsion form have satisfactory freshness but poor application properties with a relatively short application time, low degree of slip and a friction sensation perceived at the end of application.

Moreover, it is increasingly sought to have "nomad" cosmetic compositions that are easy to apply, in particular compact foundations.

The compacts present on the market today are anhydrous formulas or inverse emulsions, which have only moderate freshness. One of the disadvantages with this type of product is that, because they include a substantial quantity of fatty bodies, they leave on the skin a greasy, heavy feeling and are difficult to spread.

In addition, the presence of waxes, which contributes to the solid nature of such a composition, has for disadvantage to render the makeup result dull and matte, which corresponds increasingly less to the desires of women who want on the contrary brightness and a natural result.

EP 0 970 692 relates to the use of associative polyurethane in a cosmetic composition thickened type and associative polyurethane used for the manufacture of a dermatological composition of viscous consistency of the emulsion type, including oil-in-water.

FR 2 985 174 relates to cosmetic compositions comprising: at least a first non-volatile hydrocarbon oil selected from: the alcohols C10-C26, preferably monoalcohols, at least one hydrophilic gelling agent selected from non-ionic associative polymers of polyurethane type, preferably polyethylene oxide.

Therefore, there is currently a need for a compact foundation type formulation in the form of an oil-in-water emulsion having satisfactory application properties on the skin, as well as good cosmetic properties.

The aim of the present invention is thus to provide oil-in-water emulsions with satisfactory cosmetic application properties.

This invention also has for purpose to provide oil-in-water emulsions, in particular in the form of a compact cream, having a surface that smooths again giving the impression of having a new compact at each application.

This invention also has for purpose to provide oil-in-water emulsions, in particular in the form of a compact cream, having substantial freshness on application without the impression of heaviness.

This invention also has for purpose to provide oil-in-water emulsions, in particular in the form of a compact cream with slip, and having a very natural makeup result, which in addition does not accentuate the relief of the skin.

The present invention relates to a composition, in the form of an oil-in-water emulsion, containing:
- at least one associative polyurethane complying with the following formula (I): 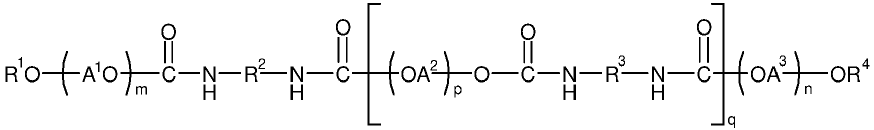 in which:
   * R¹ and R⁴ represent, independently of each other, a linear or branched bivalent hydrocarbon radical, comprising 8 to 36 carbon atoms,
   * A¹, A² and A³ represent, independently of each other, a linear or branched alkylene radical, having 2 to 4 carbon atoms;
   * m and n represent, independently of each other, an integer between 0 and 1,000,
   * p represents an integer between 5 and 500,
   * q represents an integer between 1 and 8,
   * R² and R³ represent, independently of each other, a linear or branched bivalent hydrocarbon radical, comprising 1 to 30 carbon atoms,
- at least one nonionic surfactant, said nonionic surfactant being chosen in the group consisting of: alkylpolyglucosides, gemini surfactants, silicone surfactants, and mixtures thereof;
- at least one fatty alcohol comprising a fatty chain containing at least 20 carbon atoms, and being chosen in the group consisting of: behenyl alcohol, arachidylic alcohol, and mixtures thereof;
- and at least one pigment chosen from among pigment particles coated by at least one lipophilic compound, wherein the coated pigment particles are iron oxide and/or titanium oxide particles coated with a lipophilic compound chosen from the group consisting of disodium stearoyl glutamate, isopropyl trisostearyl titanate, dimethicone, triethoxy caprylylsilane, hydrogenated lecithin and mixtures thereof,
wherein the content of associative polyurethane active substance having formula (I) is strictly greater than 1.5% by weight in relation to the total weight of said composition.

The compositions according to the invention are therefore highly structured direct emulsions with interfaces by fatty alcohols or acids and which contain in the aqueous phase an associative polyurethane.

This invention therefore relates to a makeup composition comprising a suitable associative polyurethane, a nonionic surfactant, a suitable fatty alcohol or fatty acid and pigments.

The compositions according to the invention have a clean aspect which is obtained thanks to substantial freshness on application.

In addition to this clean aspect, the application of these compositions has much slip and the makeup result obtained is very natural. In addition, these compositions do not accentuate the relief of the skin.

According to one preferred embodiment, the compositions according to the invention comprise 1.6% to 10% by weight, preferentially 2% to 8% by weight, and more preferentially 2.5% to 6% by weight, of associative polyurethane active substance having formula (I) in relation to the total weight of said composition.

According to one embodiment, the compositions according to the invention are in the form of compacts. These compositions have in particular a cream compact texture that can be defined by the fact that it has a high viscosity.

### Associative polyurethane

According to the present invention, the term "associative polyurethane" denotes an amphiphilic polymer capable, in an aqueous medium, of reversibly associating with itself or with other molecules. It generally comprises in the chemical structure thereof at least one hydrophilic region or group and at least one hydrophobic region or group.

Associative polyurethanes are non-ionic sequenced copolymers comprising in the chain, both hydrophilic sequences generally polyoxyethylenated in nature and hydrophobic sequences which may be aliphatic chains alone and/or cycloaliphatic and/or aromatic chains.

In particular, these polymers comprise at least two hydrocarbon lipophilic chains, having 8 to 36 carbon atoms, separated by a hydrophilic sequence, the hydrocarbon chains may be pendant chains or hydrophilic sequence end chains. In particular, one or a plurality of pendant chains may be envisaged. Moreover, the polymer may comprise a hydrocarbon chain at one end or at both ends of a hydrophilic sequence.

The polymers may be sequenced in triblock or multiblock form. The hydrophobic sequences may thus be at each end of the chain (for example: triblock copolymer having hydrophilic central sequence) or distributed both at the end and in the chain (multisequenced copolymer for example). The polymers may also be grafted or star polymers.

Preferentially, the associative polyurethanes according to the invention are triblock copolymers wherein the hydrophilic sequence is a polyoxyethylenated chain comprising 5 to 1,000, particularly 5 to 500, oxyethylenated groups; and comprising at least two hydrocarbon lipophilic chains having 8 to 36 carbon atoms, separated by said hydrophilic sequence, said hydrocarbon lipophilic chains may be pendant chains or hydrophilic sequence end chains.

Preferably, the associative polyurethanes according to the invention have a mean molecular weight by mass (Mw) less than or equal to 500,000 g/mol, more preferably less than or equal to 100,000 g/mol.

The compositions according to the invention comprise an associative polyurethane having formula (I) as defined above. They may also comprise a plurality of associative polyurethanes as defined above.

According to one embodiment, in formula (I), m and n represent, independently of each other, an integer between 35 and 500.

According to an embodiment, in formula (I) as defined above, R¹ and R⁴ represent, independently of each other, a linear hydrocarbon radical comprising 8 to 36 carbon atoms, i.e. a linear radical consisting of carbon atoms and hydrogen atoms.

The term "hydrocarbon radical" denotes a radical comprising only carbon and hydrogen atoms, and optionally comprising one or a plurality of unsaturations.

According to an embodiment, in formula (I), the terminal groups R¹ and R⁴ are linear chains, which may be chosen for example from the alkyl, alkenyl, or alkynyl groups.

According to an embodiment, in formula (I) as defined above, R¹ and R⁴ are hydrocarbon linear radicals comprising 8 to 30 carbon atoms.

According to one preferred embodiment, R¹ and R⁴ are linear alkyl groups comprising 1 to 30 carbon atoms. Preferentially, in formula (I), R¹ and R⁴ represent, independently of each other, a linear alkyl group comprising 8 to 30 carbon atoms.

According to one embodiment, in formula (I), R¹ and R⁴ represent a linear alkyl group comprising 18 carbon atoms.

According to an embodiment, in formula (I) as defined above, R¹ and R⁴ are hydrocarbon linear or branched radicals comprising 8 to 36, preferably 8 to 30, carbon atoms.

According to one preferred embodiment, R¹ and R⁴ are linear or branched alkyl groups comprising 8 to 36 carbon atoms. Preferentially, in formula (I), R¹ and R⁴ represent, independently of each other, a linear or branched alkyl group comprising 8 to 30 carbon atoms.

According to an embodiment, in formula (I), R¹ and R⁴ represent, independently of each other, a linear or branched alkyl group comprising 12 to 24 carbon atoms.

In formula (I), each of A¹, A² and A³ is an alkylene divalent radical which may be linear or branched, comprising 2, 3 or 4 carbon atoms. These alkylene radicals may be ethylene, propylene or butylene groups such as isobutylene.

According to an embodiment, in formula (I), each of radicals A¹, A² and A³ represents an ethylene radical.

According to one embodiment, in formula (I), m and n represent, independently of each other, an integer between 50 and 200. According to one particular embodiment, m=n=100.

According to one embodiment, in formula (I), m and n represent, independently of each other, an integer between 1 and 500, preferably between 10 and 200. According to one particular embodiment, m=n=20.

According to one embodiment, in formula (I), p represents an integer between 50 and 200. According to one particular embodiment, in formula (I), p=136.

According to one embodiment, in formula (I), p represents an integer between 10 and 500. According to one particular embodiment, in formula (I), p=240.

According to one embodiment, in formula (I), q represents an integer between 1 and 3. Preferably, in formula (I), q=1.

In formula (I), R² and R³ represent, independently of each other, a linear or branched bivalent hydrocarbon radical, corresponding to a diisocyanate residue having the respective formulas OCN-R²-NCO and OCN-R³-NCO.

These radicals comprise 1 to 30 carbon atoms, preferably 6 to 20 carbon atoms.

In particular, the radicals R² and R³ may be aliphatic, alicyclic or aromatic radicals.

According to one preferred embodiment, the radicals R² and R³ are the residues of the diisocyanates R²-(NCO)₂ and R³-(NCO)₂, these diisocyanates being chosen from aliphatic diisocyanates, aromatic diisocyanates, alicyclic diisocyanates, biphenyl diisocyanates, and phenylmethane diisocyanates.

Of the preferred diisocyanates, particular mention may be made of the following compounds: 2,4-tolylene diisocyanate, 2,6-tolylene diisocyanate, hexamethylene diisocyanate, 4,4'-diphenylmethane diisocyanate, 4,4'-diphenyl diisocyanate, 1,4-phenylene diisocyanate, and more particularly the following diisocyanates: trimethyl hexamethylene diisocyanate, isophorone diisocyanate, methylene-bis-(4-cyclohexyl)-diisocyanate, and more preferentially 1,6-hexamethylene diisocyanate (HDI).

Preferably, in formula (I), R² and R³ are hexamethylene radicals. According to this embodiment, the corresponding diisocyanate is thus hexamethylene diisocyanate (HDI).

Within the scope of the present invention, the preferred associative polyurethane is Rheolate FX1100® supplied by ELEMENTIS. This polyurethane is a polycondensate of polyethylene glycol having 136 moles of ethylene oxide, polyoxyethylenated stearyl alcohol having 100 moles of ethylene oxide and hexamethylene diisocyanate (HDI)(INCI name: PEG- 136/Steareth-100I/HDI Copolymer).

As the associative polyurethane according to the invention, mention may also be made of an associative polyurethane suitable for being obtained by means of polycondensation of at least three compounds comprising (i) at least one polyethylene glycol comprising 150 to 180 moles of ethylene oxide, (ii) stearyl alcohol or decyl alcohol and (iii) at least one diisocyanate.

Such polymers are particularly supplied by ROHM & HAAS under the names Aculyn 46 ® and Aculyn 44 ®.

ACULYN 46® is a polycondensate of polyethylene glycol having 150 or 180 moles of ethylene oxide, stearyl alcohol and methylene bis(4-cyclohexyl-isocyanate) (SMDI) at 15% by weight in a matrix of maltodextrin (4%) and water (81%)(INCI name: PEG-150/STEARYL ALCOHOL/SMDI COPOLYMER).

ACULYN 44® is a polycondensate of polyethylene glycol having 150 or 180 moles of ethylene oxide, decyl alcohol and methylene bis(4-cyclohexyl-isocyanate) (SMDI), at 35% by weight in a matrix of propylene glycol (39%) and water (26%)(INCI name: PEG-150/DECYL ALCOHOL/SMDI COPOLYMER).

As associative polyurethane according to the invention, mention may also be made of polycondensate of polyethylene glycol having 240 moles of ethylene oxide, bis-decyltetradeceth-20 ether and hexamethylene diisocyanate (HDI)(INCI name: PEG-240/HDI COPOLYMER BIS-DECYLTETRADECETH-20 ETHER), sold under the trade name Adekanol GT-700 by Shiseido.

As associative polyurethane according to the invention, mention may also be made of the polymer Polyurethane-39, sold under the trade name Luvigel Star by BASF.

In particular, suitable associative polyurethanes are those described in applications WO2009/135857 and EP 1 013 264.

### Fatty alcohol or acid

The compositions according to the invention comprise at least one fatty alcohol comprising at least 20 carbon atoms, preferably at least 22 carbon atoms, as defined above.

The present application also discloses compositions as defined above that can comprise one or more fatty alcohols, one or more fatty acids, but also mixtures of fatty alcohol(s) and fatty acid(s).

Among the fatty acids, mention can for example be made of fatty acids that comprise from 20 to 30 carbon atoms.

As particular examples of fatty acids that can be used, particular mention can be made of behenic, erucic, arachidylic acid and mixtures thereof.

Among the fatty acids, mention can for example be made of fatty acids that comprise from 20 to 30 carbon atoms, and preferably from 22 to 30 carbon atoms.

Fatty alcohol comprising from 20 to 30 carbon atoms means any pure fatty alcohol, whether or not saturated, whether or not branched, comprising 20 to 30 carbon atoms.

As examples of fatty alcohols, mention can be made of linear or branched fatty alcohols, or natural such as for example alcohols coming from plant substances (coconut, palm, etc.). Of course, other long-chain alcohols can also be used, such as for example ether-alcohols or so-called Guerbet alcohols. Finally, certain more or less long cuts of alcohols of natural origin, or compounds of the diol or cholesterol type, can also be used.

Preferably a fatty alcohol comprising from 20 to 26 carbon atoms, preferably from 20 to 24 carbon atoms, and more preferentially 22 carbon atoms is used.

As particular examples of fatty acids that can be used, particular mention can be made of behenic, erucic, arachidylic acid and mixtures thereof.

According to one embodiment, the compositions according to the invention comprise 0.1% to 10%, preferably 0.5% to 5%, by weight of fatty acid(s) or fatty alcohol(s), with respect to the total weight of said composition.

According to one preferred embodiment, the compositions according to the invention comprise behenyl alcohol. According to an embodiment, behenyl alcohol can be used alone or in a mixture with other compounds.

According to one preferred embodiment, the compositions according to the invention comprise arachidylic alcohol. According to an embodiment, behenyl alcohol can be used alone or in a mixture with other compounds. According to one preferred embodiment, the compositions of the invention comprise both behenyl alcohol and arachidylic alcohol.

### Nonionic surfactant

The compositions according to the invention comprise at least one nonionic surfactant as defined above. The present application also discloses compositions that can comprise a nonionic surfactant or a mixture of different nonionic surfactants, said nonionic surfactants being chosen in particular from alkyl- and polyalkyl- esters of poly(ethylene oxide), oxyethylene alcohols, alkyl- and polyalkyl- ethers of poly(ethylene oxide), alkyl- and polyalkyl- esters of sorbitan, polyoxyethylenated or not, alkyl- and polyalkyl- ethers of sorbitan, polyoxyethylenated or not, alkyl- and polyalkyl- glycosides or polyglycosides, in particular alkyl- and polyalkyl-glucosides or polyglucosides, alkyl- and polyalkyl-esters of sucrose, alkyl- and polyalkyl- esters of glucose, alkyl- and polyalkyl- esters of glycerol, polyoxyethylenated or not, alkyl- and polyalkyl-ethers of glycerol, polyoxyethylenated or not, gemini surfactants, silicone surfactants, cetyl alcohol and stearyl alcohol, and mixtures thereof.
1) As alkyl- and polyalkyl- esters of poly(ethylene oxide), those having a number of ethylene oxide (EO) patterns ranging from 2 to 200 are preferably used. Mention can for example be made of the stearate product PEG-8, which is sold under the trade name Myrj S8 SO by Croda, stearate 40 OE, stearate 50 OE, stearate 100 OE, laurate 20 OE, laurate 40 OE, distearate 150 OE.
2) As alkyl- and polyalkyl- esters of poly(ethylene oxide), those having a number of ethylene oxide (EO) patterns ranging from 2 to 200 are preferably used. Mention can for example be made of cetyl ether 23 OE, oleyl ether 50 OE, phytosterol 30 OE, steareth 40, steareth 100, beheneth 100.
3) As oxyalkylene alcohols, in particular oxyethylenated and/or oxypropylenated, those that contain from 1 to 150 oxyalkylene and/or oxypropylene units are preferably used, in particular containing from 20 to 100 oxyalkylene units, in particular ethoxylated fatty alcohols, particularly C₈-C₂₄ and particularly C₁₂-C₁₈, which can be ethoxylated or not ethoxylated, for example ethoxylated stearyl alcohol with 20 oxyalkylene units (INCI name Steareth-20), for example Brij 78 sold by Uniqema, ethoxylated cetearyl alcohol with 30 oxyalkylene units (INCI name: Ceteareth-30), and mixtures of fatty C₁₂-C₁₅ alcohols comprising 7 oxyethylene units (INCI name: C₁₂-C₁₅ Pareth-7), for example the product sold under the name Neodol 25-7® by Shell Chemicals; or in particular oxyalkylene alcohols (oxyethylenated and/or oxypropylenated) containing from 1 to 15 oxyethylene and/or oxypropylene units, in particular C₈-C₂₄ ethoxylated fatty alcohols, and preferably C₁₂-C₁₈ fatty alcohols, such as ethoxylated stearyl alcohol with two oxyethylene units (INCI name: Steareth-2), for example the product Brij 72 sold by Uniqema.
4) As alkyl- and polyalkyl- esters of sorbitan, polyoxyethylenated or not, those having a number of ethylene oxide (EO) patterns ranging from 0 to 100 are preferably used. Mention can for example be made of sorbitan laurate 4 or 20 OE, in particular polysorbate 20 (or polyoxyethylene (20) sorbitan monolaurate) such as the product Tween 20 sold by Uniqema, sorbitan palmitate 20 OE, sorbitan stearate 20 OE, sorbitan oleate 20 OE or Cremophors (RH 40, RH 60 ...) from BASF. Mention can also be made of the mixture of sorbitan stearate and of sucrose cocoate (sold under the name Arlacel 2121U-FL by Croda).
5) As alkyl- and polyalkyl- ethers of sorbitan, polyoxyethylenated or not, those having a number of ethylene oxide (EO) patterns ranging from 0 to 100 are preferably used.
6) As alkyl- and polyalkyl- glucosides or polyglucosides, those containing an alkyl group comprising from 6 to 30 carbon atoms and preferably from 6 to 18, even from 8 to 16 carbon atoms are preferably used, and containing a glucoside group comprising preferably from 1 to 5, in particular 1, 2 to 3 glucoside units. The alkylpolyglucosides can be chosen for example from decylglucoside (Alkyl-C₉/C₁₁-polyglucoside (1.4)) such as the product sold under the trade name Mydol 10® by Kao Chemicals or the product sold under the trade name Plantacare 2000 UP® by Henkel and the product sold under the trade name ORAMIX NS 10® by SEPPIC; caprylyl/capryl glucoside such as the product sold under the trade name Plantacare KE 3711® by Cognis or ORAMIX CG 110® by SEPPIC; laurylglucoside such as the product sold under the trade name Plantacare 1200 UP® by Henkel or Plantaren 1200 N® by Henkel; cocoglucoside such as the product sold under the trade name Plantacare 818 UP® by Henkel; caprylylglucoside such as the product sold under the trade name Plantacare 810 UP® by Cognis; the mixture of arachidyl glucoside and of behenyl alcohol and arachidylic alcohol, of which the INCI name is Arachidyl alcohol (and) behenyl alcohol (and) arachidyl glucoside, sold under the trade name Montanov 202 by Seppic; cetearylglucoside, sold under the trade name Tego care CG 90 by Evonik Goldschmidt; methyl glucose sesquistearate sold under the trade name Glucate SS emulsifier by Lubrizol; the mixture of C₁₂-C₂₀ alkylglucoside and of C₁₄-C₂₂ alcohol (INCI name: C14-C22 alcohol (and) C12-C20 alkylglucoside) sold under the trade name Montanov L by Seppic; and mixtures thereof.
7) As alkyl- and polyalkyl- esters of sucrose, in particular C₁₂-C₂₆ alkyl esters, mention can for example be made of sucrose stearate, sold in particular under the trade name Tegosoft PSE 141 G by Evonik Goldschmidt; the mixture of sorbitan stearate and of sucrose cocoate (sold under the trade name Arlatone Arlacel 2121 U-FL by Croda), Crodesta F150, sucrose monolaurate sold under the trade name Crodesta SL 40, products sold by Ryoto Sugar Ester such as for example, sucrose palmite sold under the reference Ryoto Sugar Ester P1670, Ryoto Sugar Ester LWA 1695, Ryoto Sugar Ester 01570.
8) As alkyl- and polyalkyl- esters of glycerol, polyoxyethylenated or not, those having a number of ethylene oxide (EO) patterns ranging from 0 to 100 and a number of glycerol patterns ranging from 1 to 30 are preferably used. Mention can for example be made of PEG-150 distearate sold under the trade name Kessco PEG 6000 DS by Italmatch Chemicals Arese; hexaglyceryl monolaurate; the mixture of glyceryl stearate and of PEG-100 stearate of which the INCI name is glyceryl stearate (and) PEG-100 stearate, sold under the trade name Tego Care 180 by Evonik Goldscmidt or under the trade name Arlacel 165-FL-(CQ) by Croda or under the trade name Simulsol 165 by Seppic; the mixture of methylglucose distearate and of polyglyceryl (INCI name: polyglyceryl-3 methylglucose distearate) sold under the trade name Tego Care 450 by Evonik Goldschmidt; polyglycerol citric and stearic ester (INCI name: polyglyceryl-3 dicitrate/stearate) sold under the trade name Tego Care PSC 3 by Evonik Goldschmidt and PEG-30 glyceryl stearate.
9) As alkyl- and polyalkyl- ethers of glycerol, polyoxyethylenated or not, those having a number of ethylene oxide (EO) patterns ranging from 0 to 100 and a number of glycerol patterns ranging from 1 to 30 are preferably used. For example, mention can be made of Nikkol Batyl alcohol 100, Nikkol chimyl alcohol 100.
10) cetylic alcohol and stearylic alcohol;
11) gemini surfactants that can be mentioned include the mixture of sodium dilauramidoglutamide lysine and of butyleneglycol (INCI name: sodium dilauramidoglutamide lysine) sold under the trade name Pellicer LB -30G by Asahi Kasei Chemicals. Gemini surfactants can also comply with the following formula (I): 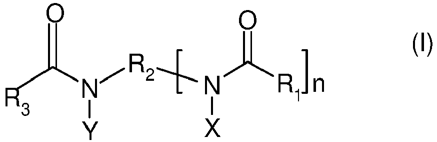 wherein:
   - R¹ and R³ represent, independently of each other, an alkyl radical, comprising 1 to 25 carbon atoms;
   - R² represents a spacer group consisting of a linear or branched alkylene chain containing from 1 to 12 carbon atoms;
   - X and Y represent, independently of each other, a -(C₂H₄O)ₐ-(C₃H₆O)_{b}Z group, wherein:
      - Z represents a hydrogen atom or a radical -CH₂-COOM, -SO₃M, -P(O)(OM)₂, -C₂H₄-SO₃M, -C₃H₆-SO₃M or -CH₂(CHOH)₄CH₂OH, wherein M and M' represent H or an alkali metal or alkali earth metal ion or an ammonium or alkanolammonium ion,
      - a varies from 0 to 15,
      - b varies from 0 to 10, and
      - the sum of a + b varies from 1 to 25; and
   - n varies from 1 to 10.

The gemini surfactant of formula (I) is preferably such that each one of the R₁-CO- and R₃-CO- groups comprises from 8 to 20 carbon atoms, and preferably represents a residue of coconut fatty acid (comprising mainly lauric acid and myristic acid).

In addition, this surfactant is preferably such that, for each one of the radicals X and Y, the sum of a and b has an average value that varies from 10 to 20 and is preferably equal to 15. A preferred group for Z is the -SO₃M group, where M is preferably an alkali metal ion such as the sodium ion.

The spacer R₂ consists advantageously in a C₁-C₃ linear alkylene chain and preferably an ethylene chain (CH₂CH₂).

Finally, n is advantageously equal to 1.

A surfactant of this type is in particular the one identified by the INCI name: Sodium dicocoylethylenediamine PEG-15 sulfate, having the following structure: 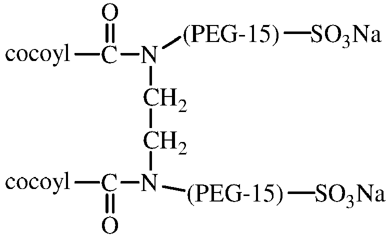 with the understanding that PEG represents the CH₂CH₂O group and that 'cocoyl' represents the coconut fatty acid residue.

This surfactant has a molecular structure that is very similar to that of ceramide-3.

Preferably, the gemini surfactant according to the invention is used in a mixture with other surfactants, and in particular in a mixture with (a) an ester of a C₆-C₂₂ fatty acid (preferably C₁₄-C₂₀ such as stearate) and of glyceryle, (b) a diester of a C₆-C₂₂ fatty acid (preferably C₁₄-C₂₀ such as stearate) and of citric acid and glycerol (in particular a C₆-C₂₂ fatty acid ester and glyceryl monocitrate), and (c) a C₁₀-C₃₀ fatty alcohol (preferably behenyl alcohol). Advantageously, the composition according to the invention comprises a mixture of sodium dicocoylethylenediamine PEG-15 sulfate, of glyceryl stearate, of glyceryl stearate monocitrate and of behenyl alcohol. Preferentially, the gemini surfactant according to the invention represents from 10% to 20% by weight, and advantageously 15% by weight; the ester of the C₆-C₂₂ fatty acid and glyceryle represents from 30% to 40% by weight, advantageously 35% by weight; the diester of the C₆-C₂₂ fatty acid and of citric acid and of glycerol represents from 10% to 20% by weight, advantageously 15% by weight; and the C₁₀-C₃₀ fatty alcohol represents from 30% to 40% by weight, advantageously 35% by weight, in relation to the total weight of the surfactant mixture containing the gemini surfactant.

Advantageously, the composition according to the invention comprises a mixture of 10% to 20% by weight of sodium dicocoylethylenediamine PEG-15 sulfate, from 30% to 40% (in particular 35%) by weight of glyceryl stearate, from 10% to 20% (in particular 15%) by weight of glyceryl stearate monocitrate and from 30% to 40% (in particular 35%) by weight of behenyl alcohol, in relation to the total weight of the surfactant mixture containing the gemini surfactant.

For example, the gemini surfactant can be used as a mixture with other surfactants in the form of products sold by Sasol under the trade name Ceralution®, such as in particular the following products:
- Ceralution® H: Behenyl Alcohol, Glyceryl Stearate, Glyceryl Stearate Citrate and Sodium Dicocoylethylenediamine PEG-15 Sulfate (INCI name),
- Ceralution® F: Sodium Lauroyl Lactylate and Sodium Dicocoylethylenediamine PEG-15 Sulfate (INCI name),
- Ceralution® C: Aqua, Capric/Caprylic triglyceride, Glycerin, Ceteareth-25, Sodium Dicocoylethylenediamine PEG-15 Sulfate, Sodium Lauroyl Lactylate, Behenyl Alcohol, Glyceryl Stearate, Glyceryl Stearate Citrate, Gum Arabic, Xanthan Gum, Phenoxyethanol, Methylparaben, Ethylparaben, Butylparaben, Isobutylparaben (INCI name).

The gemini surfactant represents from 3% to 50% by weight of these mixtures. Preferably, the composition comprises, as a gemini surfactant, the compound with INCI name: Behenyl alcohol, glyceryl stearate, glyceryl stearate citrate and sodium dicocoylethylenediamine PEG-15 sulfate, sold under the trade nameCeralution® H by Sasol.

12) the term "silicone surfactant" designates a silicone compound comprising at least one oxyalkylenated chain, in particular comprising at least one oxyalkylenated (-OCH₂CH₂-) and/or oxypropylenated (-OCH₂CH₂CH₂-) chain and or a polyglycerolated chain. As silicone surfactant, the examples of polydimethylsiloxanes comprising both oxyethylenated groups and oxypropylenated groups can be mentioned. Mention can for example be made of polydimethylsiloxane with a oxyethylene/oxypropylene termination sold as a mixture with triglycerides of caprylic/capric acid sold under the trade name Abil care 85 by Evonik Goldschmidt (INCI name: BIS-PEG/PPG-16/16 PEG/PPG-16/16 Dimethicone/Caprylic/Capric Triglyceride), with polydimethylsiloxane comprising an alpha-omega polyether group (OE/OP: 40/60), sold under the trade name Abil B8832 by Goldschmidt (INCI name: BIS-PEG/PPG-20/20 Dimethicone), polydimethylsiloxane oxyethylene oxypropylene sold under the trade name Abil B88184 by Goldschmidt (INCI name: PEG/PPG-20/6 Dimethicone), polydimethylsiloxane with oxyethylene/oxypropylene termination, sold under the trade name Abil Care XL 80 by Evonik Goldscmidt (INCI name: BIS-PEG/PPG-20/5 PEG/PPG20/5 Dimethicone; Methoxy PEG/PPG-25/4) Dimethicone; caprylic/capric triglyceride and polydimethyl/methylsiloxane oxyethylene oxypropylene sold under the trade name Abil B8852 by Goldschmidt (INCI name: PEG/PPG-4/12 Dimethicone), and mixtures thereof. Alternatively, the silicone surfactant can also be a polydimethylsiloxane with polyglycerylated chains, such as polyglyceryl-3 polymethylsiloxyethyl dimethicone, laurylpolyglyceryl-3 polymethylsiloxyethyl dimethicone and polyglyceryl-3 disiloxane dimethicone and mixtures thereof. Such silicone surfactants are described in patent application EP 1 213 316. According to a particular embodiment, the silicone surfactant is a polyglyceryl-3 disiloxane dimethicone sold under the trade name KF 6100 by Shin Etsu.

13) and mixtures thereof.

According to the invention, the fatty alcohol such as mentioned hereinabove can be included in the nonionic surfactant, with the latter corresponding to a mixture of different compounds among which one or several fatty alcohols.

As such, mention can be made in particular of the mixture of behenyl alcohol and of arachidic alcohol, of which the INCI name is ARACHIDYL ALCOHOL (and) BEHENYL ALCOHOL (and) ARACHIDYL GLUCOSIDE, sold under the trade name Montanov 202 by Seppic.

Mention can also be made of the mixture of behenyl alcohol, of glyceryl stearate, of disulfate of disodium ethylene dicocamide PEG-15 and of glyceryl stearate and citrate, of which the INCI name is BEHENYL ALCOHOL (and) GLYCERYL STEARATE (and) DISODIUM ETHYLENE DICOCAMIDE PEG-15 DISULFATE (and) GLYCERYL STEARATE CITRATE, sold under the trade name Ceralution H by Sasol.

According to an embodiment, the compositions of the invention comprise at least one silicon surfactant, and preferably mention can for example be made of polydimethylsiloxane with a oxyethylene/oxypropylene termination sold as a mixture with triglycerides of caprylic/capric acid sold under the trade name Abil care 85 by Evonik Goldschmidt (INCI name: BIS-PEG/PPG-16/16 PEG/PPG-16/16 Dimethicone/Caprylic/Capric Triglyceride).

According to one embodiment, the compositions according to the invention comprise at least sucrose stearate.

According to one embodiment, the compositions according to the invention comprise 0.5% to 10%, preferably 2% to 8% by weight of nonionic surfactants with respect to the total weight of said composition.

### Pigment

The compositions according to the invention comprise at least one pigment. They can therefore comprise a mixture of identical or different pigments.

The term "pigments" should be understood to mean white or colored, mineral or organic particles, which are insoluble in an aqueous solution and are intended for coloring and/or opacifying the resulting film.

According to one embodiment, the pigment content is 3% to 30%, preferably 5% to 20%, and more preferably 5% to 16%, by weight in relation to the total weight of said composition.

As mineral pigments suitable for use in the invention, mention may be made of titanium, zirconium or cerium oxides, and also zinc, iron or chromium oxides, ferric blue, manganese violet, ultramarine blue and chromium hydrate. Preferably, the composition according to the invention includes at least titanium oxides and iron oxides.

Of the organic pigments suitable for use in the invention, mention may be made of carbon black, D & C type pigments, lacquers based on cochineal carmine, barium, strontium, calcium, aluminum or diketopyrrolopyrrole (DPP) described in the documents EP 0 542 669, EP 0 787 730, EP 0 787 731 and WO 96/08537.

### Coated pigment and/or nacre particles

The present application also discloses compositions that can further comprise pigment and or nacres particle(s) coated with a lipophilic compound, these particles may be identical or different. These compositions may thus comprise mixtures of pigment and/or nacre particles of different types.

The compositions according to the invention comprise pigment particles coated with a lipophilic compound as defined above.

The present application also discloses a composition that can comprise at least one pigment and/or one nacre coated with at least one lipophilic compound, preferably at least one pigment coated with at least one lipophilic compound.

The pigments and/or nacres are coated hydrophobically so as to be situated in the oil phase of the emulsion, i.e. in the internal phase.

Preferably, the composition comprises at least inorganic pigments coated with at least one lipophilic compound, particularly at least titanium oxide and iron oxides coated with at least one lipophilic compound.

The coating of a pigment and/or a nacre according to the present disclosure generally denotes the full or partial surface treatment of the pigment and/or nacre by a surface agent, absorbed, adsorbed or grafted onto said pigment.

The coating of the pigment and/or nacre particles may be partial or full. Within the present disclosure, the term "partial coating" denotes that the pigment and/or nacre is coated with at least one coating layer at a rate of 50% to 99.9% of the surface of said pigment (or said nacre).

The coating of the pigments or nacres may comprise one or a plurality of lipophilic compounds, and optionally also one or a plurality of non-lipophilic compounds.

The coated pigments or nacres are obtained by means of full or partial surface treatment of the pigment or nacre with a lipophilic compound (or surface agent). This surface agent is thus absorbed, adsorbed or grafted onto said pigments/said nacres.

The surface-treated pigments or nacres may be prepared using chemical, electronic, mechano-chemical or mechanical surface treatment techniques well known to those skilled in the art. Commercial products may also be used.

The surface agent may be absorbed, adsorbed or grafted onto the pigments or nacres by means of solvent evaporation, chemical reaction or creation of a covalent bond.

According to one alternative embodiment, the surface treatment consists of a solid particle coating.

The coating may represent 0.1% to 20% by weight and particularly 0.5% to 5% by weight of the total weight of the coated pigment or nacre.

The coating may be carried out for example by adsorbing a liquid surface agent on the surface of the solid particles merely by mixing while stirring the particles and said surface agent, optionally heated, before incorporating the particles in the other ingredients of the makeup or treatment composition.

The coating may be carried out for example by means of a chemical reaction of a surface agent with the surface of the solid pigment particles and the creation of a covalent bond between the surface agent and the particles. This method is particularly described in the patent US 4 578 266.

The chemical surface treatment may consist of diluting the surface agent in a volatile solvent, dispersing the pigments in this mixture, and then slowly evaporating the volatile solvent, such that the surface agent is deposited on the surface of the pigments.

According to an embodiment, when the pigment and/or nacre particles are coated with an amino acid, in particular by an acetylated amino acid, such as lipophilic compounds, this surface treatment implies the creating of covalent bonds and allows these coated particles to be dispersed in the oily phase.

### Lipophilic or hydrophobic treatment agent

According to one particular embodiment, the pigments or nacres may be coated with at least one lipophilic compound chosen from the group consisting of silicone surface agents, fluorinated surface agents, fluorosilicone surface agents, metallic soaps, N-acylated amino acids and salts thereof, lecithin and derivatives thereof, isopropyl triisostearyl titanate, isostearyl sebacate, plant or animal natural waxes, polar synthetic waxes, fatty esters, phospholipids and mixtures thereof.

### Silicone surface agent

According to one particular embodiment, the pigments or nacres may be fully or partially surface-treated with a silicone compound.

The silicone surface agents may be chosen from organopolysiloxanes, silane derivatives, silicon-acrylate copolymers, silicone resins, and mixtures thereof.

The term organopolysiloxane denotes a compound having a structure comprising an alternation of silicon atoms and oxygen atoms and comprising organic radicals bound with silicon atoms.

As examples of pigments treated with a silicone compound, mention may be made of the following pigments treated with:
- Triethoxycaprylylsilane treatment such as the AS surface treatment (LCW) and the OTS surface treatment (Daito);
- Methicone treatment such as the SI surface treatment (LCW);
- Dimethicone treatment such as the Covasil 3.05 (LCW) or SA (Miyoshi) or SI01 (Daito) surface treatment
- Dimethicone / Trimethylsiloxysilicate treatment such as the Covasil 4.05 surface treatment (LCW); and
- Acrylate Copolymer / Dimethicone treatment such as the ASC surface treatment (Daito).

### Fluorinated surface agent

The pigments or nacres may be fully or partially surface-treated with a fluorinated compound.

The fluorinated surface agents may be chosen from perfluoroalkyl phosphates, perfluoropolyethers, polytetrafluoropolyethylene (PTFE), perfluoroalkanes, perfluoroalkyl silazanes, hexafluoropropylene polyoxides or polyorganosiloxanes comprising perfluoroalkyl perfluoropolyether groups.

As examples of commercial pigments treated with a fluorinated compound, mention may be made of the following pigments treated with:
- Perfluoropolymethylisopropyl ether treatment such as the FHC surface treatment (LCW); and
- Perfluoroalkyl Phosphate treatment such as the PF surface treatment (Daito).

### Fluorosilicone surface agent

The pigments or nacres may be fully or partially surface-treated with a fluorosilicone compound.

The fluorosilicone compound may be chosen from perfluoroalkyl dimethicones, perfluoroalkyl silanes and perfluoroalkyl trialkoxysilanes.

As examples of commercial pigments treated with a fluorosilicone compound, mention may be made of the following pigments subjected to the following treatments:
- Acrylate Copolymer / Dimethicone and Perfluoroalkyl Phosphate treatment such as the FSA surface treatment (Daito).
- Polymethyl hydrogen siloxane / Perfluoroalkyl Phosphate treatment such as the FS01 surface treatment (Daito);
- Octyltriethylsilane / Perfluoroalkyl Phosphate treatment such as the FOTS surface treatment (Daito); and
- Perfluorooctyl Triethoxysilane treatment such as the FHS surface treatment (Daito).

### Other lipophilic surface agents

The hydrophobic treatment agent may also be chosen from metallic soaps such as aluminum dimyristate, the aluminum salt of hydrogenated tallow glutamate.

As metallic soaps, mention may particularly be made of metallic soaps of fatty acids having 12 to 22 carbon atoms, and particularly those having 12 to 18 carbon atoms.

The metal of the metallic soap may particularly be zinc or magnesium. As a metallic soap, zinc laurate, magnesium stearate, magnesium myristate, zinc stearate, and mixtures thereof may be used.

The fatty acid may particularly be chosen from lauric acid, myristic acid, stearic acid and palmitic acid.

The hydrophobic treatment agent may also be chosen from N-acylated amino acids or salts thereof which may comprise an acyl group having 8 to 22 carbon atoms, such as for example a 2-ethylhexanoyl, caproyl, lauroyl, myristoyl, palmitoyl, stearoyl or cocoyl group.

The amino acid may be for example lysine, glutamic acid or alanine.

The salts of these compounds may be aluminum, magnesium, calcium, zirconium, zinc, sodium or potassium salts.

In this way, according to one particularly preferred embodiment, an N-acylated amino acid derivative may be particularly a glutamic acid derivative and/or any of the salts thereof, and more particularly a stearoyl glutamate, such as for example aluminum stearoyl glutamate.

According to one preferred embodiment, the composition according to the invention comprises pigment particles obtained by treating with aluminum stearoyl glutamate. In particular, the composition may comprise iron and/or titanium oxides coated with aluminum stearoyl glutamate.

The hydrophobic treatment agent may also be chosen from:
- lecithin and derivatives thereof,
- isopropyl triisostearyl titanate,
- isostearyl sebacate,
- plant or animal natural waxes or polar synthetic waxes,
- fatty esters, particularly with jojoba esters,
- phospholipids, and
- mixtures thereof.

As examples of commercial pigments treated with compounds as defined above, mention may be made for example of pigments subjected to the following treatments:
- Lauroyl Lysine treatment such as the LL surface treatment (LCW);
- Lauroyl Lysine Dimethicone treatment such as the LL / SI surface treatment (LCW);
- Magnesium Myristate treatment such as the MM surface treatment (LCW);
- Magnesium Stearate treatment such as the MST surface treatment (Daito).
- Hydrogenated Lecithin treatment such as the HLC surface treatment (LCW);
- Aluminum Dimyristate treatment such as the MI surface treatment (Miyoshi);
- Isostearyl Sebacate treatment such as the HS surface treatment (Miyoshi);
- Disodium Stearoyl Glutamate treatment such as the NAI surface treatment (Miyoshi);
- Sodium Dilauramidoglutamide lysine treatment such as the ASL surface treatment (Daito);
- Dimethicone / Disodium Stearoyl Glutamate treatment such as the SA / NAI surface treatment (Miyoshi);
- Hydrogenated Stearyl Olive Esters treatment such as the MiyoNAT surface treatment (Miyoshi);
- Lauroyl Lysine / Aluminum Tristearate treatment such as the LL-StAI surface treatment (Daito);
- Isopropyl Titanium Triisostearate treatment such as the ITT surface treatment (Daito); or
- Perfluoroalkyl Phosphate / Isopropyl Titanium Triisostearate treatment such as the PF + ITT surface treatment (Daito).

The pigments coated according to the invention with at least one lipophilic compound, may be present in a composition according to the invention at a concentration ranging from 1% to 30% by weight, in relation to the total weight of the composition, preferably 3% to 20% by weight.

According to the invention, the coated pigment particles are iron oxide and/or titanium oxide particles coated with a lipophilic compound chosen from the group consisting of disodium stearoyl glutamate, isopropyl trisostearyl titanate, dimethicone, triethoxy caprylylsilane, hydrogenated lecithin and mixtures thereof.

Preferably, the coated pigments used as iron oxides and titanium oxides coated with Disodium Stearoyl Glutamate (NAI), isopropyl trisostearyl titanate (ITT), Dimethicone (SA), Triethoxy caprylylsilane (AS) or Hydrogenated Lecithin (HLC).

According to an advantageous embodiment, the compositions according to the invention include iron oxide and/or titanium oxide, coated or not.

When the pigments used as such are not coated, they are present in the external phase namely the aqueous phase, and they are present in the internal phase (oily phase) if they are treated hydrophobically.

According to an embodiment, the use of coated hydrophobic pigments in the internal phase makes it possible to obtain a makeup result that is more covering, a covering which is often lacking in pigmented formulas in direct emulsion and which is an expectation of consumers of compacts.

Furthermore, the presence of hydrophobic coated pigment makes it possible to obtain a makeup result with more cover and thus closer to the makeup result obtained with a conventional water-in-oil foundation.

As the result offers more cover, facial color imperfections are toned down more, giving rise to a superior makeup result.

The compositions according to the invention are in the form of an O/W emulsion containing a dispersed fat (or oil) phase and a continuous aqueous phase.

### Aqueous phase

The aqueous phase of the compositions according to the invention comprises water.

According to one embodiment, the aqueous phase of the compositions according to the invention represents at least 40% by weight in relation to the total weight of said composition.

According to one embodiment, the compositions according to the invention comprise at least 35% by weight of water, particularly at least 40% by weight of water in relation to the total weight of said composition.

A water suitable for the invention may be a floral water such as cornflower water and/or a mineral water such as Vittel water, Lucas water or La Roche Posay water and/or a spring water.

The aqueous phase may also comprise water-miscible organic solvents (at ambient temperature - 25°C) such as for example mono-alcohols having 2 to 6 carbon atoms such as ethanol, isopropanol; polyols having particularly 2 to 20 carbon atoms, preferably having 2 to 10 carbon atoms, and preferentially having 2 to 6 carbon atoms, such as glycerol, propylene glycol, butylene glycol, pentylene glycol, hexylene glycol, caprylylglycol, dipropylene glycol, diethylene glycol; glycol ethers (having particularly 3 to 16 carbon atoms) such as mono, di- or tripropylene glycol alkyll(C₁-C₄)ether of mono, di- or tripropylene glycol, alkyl(C₁-C₄)ethers of mono, di- or triethylene glycol, and mixtures thereof.

According to one embodiment, the aqueous phase of the compositions according to the invention comprises glycerol, phenoxyethanol, or a mixture thereof.

The aqueous phase may also include any water-soluble or water-dispersible compound compatible with an aqueous phase, such as gelling agents, film-forming polymers, thickeners, surfactants and mixtures thereof.

### Oily phase

The compositions according to the invention also comprise a dispersed oil phase.

According to one embodiment, the oil phase represents 10% to 50%, and preferably 15% to 40%, by weight in relation to the total weight of said composition.

The oily phase (or fat phase) of the compositions according to the invention comprises at least one oil. It may consist of a single oil or a mixture of a plurality of oils. The term "oil" is intended to mean any fatty substance in liquid form at ambient temperature (20-25°C) and at atmospheric pressure. These oils may be of plant, mineral or synthetic origin.

According to one embodiment, the oils are chosen from the group consisting of hydrocarbon oils, silicone oils, fluorinated oils and mixtures thereof.

According to the present invention, the term "hydrocarbon oil" denotes an oil containing mainly hydrogen and carbon atoms.

The term "silicone oil" denotes an oil comprising at least one silicon atom and particularly comprising at least one Si-O group.

The term "fluorinated oil" denotes an oil comprising at least one fluorine atom.

The oils may optionally comprise oxygen, nitrogen, sulfur and/or phosphorus atoms, for example in the form of hydroxyl or acid radicals

According to one embodiment, the oily phase of the compositions according to the invention comprises at least one volatile oil and/or at least one non-volatile oil.

### Volatile oils

According to one embodiment, the oily phase of the compositions according to the invention comprises at least one volatile oil. The oily phase of the compositions according to the invention may comprise a mixture of a plurality of volatile oils.

The term "volatile oil" denotes any non-aqueous medium capable of evaporating on contact with the skin or the lips, in less than one hour, at ambient temperature and at atmospheric pressure. The volatile oil is a volatile cosmetic oil that is liquid at ambient temperature. More precisely, a volatile oil has an evaporation rate of between 0.01 and 200 mg/cm²/min, inclusive.

To measure this evaporation rate, 15g of oil or an oil mixture to be tested are introduced into a crystallizer with a diameter of 7cm, placed on a scale located in a large chamber of around 0.3m³, with controlled temperature, at 25°C, and hygrometry, at 50% relative humidity. The liquid is left to evaporate freely, without stirring, by allowing ventilation with a fan (PAPST-MOTOREN, reference 8550 N, rotating at 2700 rpm) arranged vertically above the crystallizer containing said oil or said mixture, with the blades being directed toward the crystallizer and at a distance of 20 cm with respect to the crystallizer base. The mass of oil remaining in the crystallizer is measured at regular intervals. The evaporation rates are expressed in mg of oil evaporated per unit of surface (cm²) and per unit of time (minutes).

The volatile oils may be hydrocarbon, silicone or fluorinated oils.

The volatile oils may be chosen from hydrocarbon oils having 8 to 16 carbon atoms, and particularly branched C₈-C₁₆ alkanes (also known as isoparaffins or isoalkanes), such as isododecane (also known as 2,2,4,4,6-pentamethylheptane), isodecane, isohexadecane, and, for example, the oils sold under the trade names ISOPARS® or PERMETHYLS®.

As hydrocarbon volatile oils, mention may also be made of linear C₉-C₁₇, such as dodecane (C12) and tetradecane (C14), marketed respectively under the references PARAFOL® 12-97 and PARAFOL® 14-97 (Sasol) and such as the alkanes obtained according to the method described in the international application WO2007/068371 A1, such as the mixture of undecane (C₁₁) and tridecane (C₁₃).

According to one embodiment, the oily phase of the compositions according to the invention comprises at least one volatile silicone oil.

It is also possible to use, as volatile oils, volatile silicones, such as, for example, volatile linear or cyclic silicone oils, in particular those having a viscosity below or equal to 8 centistokes (cst) (8 x 10⁻⁶ m²/s), and having, in particular, 2 to 10 silicon atoms, and in particular, 2 to 7 silicon atoms, these silicones optionally comprising alkyl or alkoxyl groups having 1 to 10 carbon atoms. It is possible to cite, as a volatile silicone oil that can be used in the invention, in particular, dimethicones with a viscosity of 5 and 6 cst, octamethyl cyclotetrasiloxane, decamethyl cyclopentasiloxane, dodecamethyl cyclohexasiloxane, heptamethyl hexyltrisiloxane, heptamethyloctyl trisiloxane, hexamethyl disiloxane, octamethyl trisiloxane, decamethyl tetrasiloxane, dodecamethyl pentasiloxane, and mixtures thereof.

More specifically, as a volatile silicone oil, mention may be made of linear or cyclic silicone oils having 2 to 7 silicon atoms, these silicones optionally comprising alkyl or alkoxyl groups having 1 to 10 carbon atoms.

Mention may be made, as a volatile fluorinated oil, for example, of nonafluoromethoxybutane or perfluoromethylcyclopentane, and mixtures thereof.

### Non-volatile oils

According to one embodiment, the oily phase of the compositions according to the invention comprises at least one non-volatile oil. The oily phase of the compositions according to the invention may comprise a mixture of a plurality of non-volatile oils.

The term "non-volatile oil" denotes an oil remaining on the skin or keratin fiber at ambient temperature and atmospheric pressure. More specifically, a non-volatile oil has an evaporation rate strictly below 0.01 mg/cm²/min.

The non-volatile oils may, in particular, be chosen from non-volatile hydrocarbon, fluorinated and/or silicone oils.

As a non-volatile hydrocarbon oil, mention may be made of:
- hydrocarbon oils of plant origin, such as phytostearyl esters, for instance phytostearyl oleate, phytostearyl isostearate and lauroyl/octyldodecyl/phytostearyl glutamate (AJINOMOTO, ELDEW PS203), triglycerides constituted of glycerol and fatty acid esters, in particular in which the fatty acids may have chain lengths ranging from C₄ to C₃₆, and in particular from C₁₈ to C₃₆, these oils may be linear or branched, saturated or unsaturated; these oils may in particular be heptanoic or octanoic triglycerides, shea oil, alfalfa oil, poppy seed oil, pumpkin oil, millet oil, barley oil, quinoa oil, rye oil, candlenut oil, passionflower oil, shea butter, aloe oil, sweet almond oil, peach kernel oil, groundnut oil, argan oil, avocado oil, baobab oil, borage oil, broccoli oil, calendula oil, camelina oil, canola oil, carrot oil, safflower oil, hemp oil, rapeseed oil, cotton seed oil, coconut oil, pumpkin seed oil, wheat germ oil, jojoba oil, lily oil, macadamia oil, corn oil, meadowfoam oil, St. John's Wort oil, monoi oil, hazelnut oil, apricot kernel oil, walnut oil, olive oil, evening primrose oil, palm oil, blackcurrant seed oil, kiwi seed oil, grape seed oil, pistachio oil, pumpkin squash oil, winter squash oil, musk rose oil, sesame oil, soybean oil, sunflower oil, castor oil and watermelon oil, and mixtures thereof, or alternatively caprylic/capric acid triglycerides, such as those sold by STEARINERIE DUBOIS or those sold under the names MIGLYOL 810®, 812® and 818® by DYNAMIT NOBEL,
- linear or branched hydrocarbons of mineral or synthetic origin, such as liquid paraffins and derivatives thereof, petroleum jelly, polydecenes, polybutenes, hydrogenated polyisobutene such as Parleam, squalane;
- synthetic ethers having 10 to 40 carbon atoms such as dicaprylether;
- synthetic esters, such as the oils having the formula R₁COOR₂, wherein R¹ represents a linear or branched fatty acid residue comprising 1 to 40 carbon atoms, and R² represents a hydrocarbon chain, particularly branched containing 1 to 40 carbon atoms provided that the sum of the number of carbon atoms of the chains R¹ and R² is greater than or equal to 10. The esters may particularly be chosen from fatty alcohol and acid esters, such as for example: Icetostearyl octanoate, isopropyl alcohol esters, such as isopropyl myristate, isopropyl palmitate, ethyl palmitate, 2-ethylhexyl palmitate, isopropyl stearate or isostearate, isostearyl isostearate, octyl stearate, hydroxylated esters, such as isostearyl lactate, octyl hydroxystearate, diisopropyl adipate, heptanoates, particularly isostearyl heptanoate, alcohol or polyalcohol octanoates, decanoates or ricinoleates, such as propylene glycol dioctanoate, cetyl octanoate, tridecyl octanoate, 2-ethylhexyl 4-diheptanoate and palmitate, alkyl benzoate, polyethylene glycol diheptanoate, propylene glycol 2-diethylhexanoate, and mixtures thereof, C₁₂-C₁₅ alkyl benzoates, hexyl laurate, neopentanoic acid esters, such as isodecyl neopentanoate, isotridecyl neopentanoate, isostearyl neopentanoate, or octyldodecyl neopentanoate, isononanoic acid esters, such as isononyl isononanoate, isotridecyl isononanoate and octyl isononanoate, hydroxylated esters such as isostearyl lactate and diisostearyl malate;
- polyol esters and pentaerythritol esters, such as dipentaerythrityl tetrahydroxystearate/tetraisostearate;
- esters of diol dimers and diacid dimers, such as Lusplan DD-DA5® and Lusplan DD-DA7®, marketed by NIPPON FINE CHEMICAL and described in application US 2004-175338;
- copolymers of a diol dimer and of a diacid dimer and esters thereof, such as copolymers of dilinoleyl diol dimers/dilinoleic dimers and esters thereof, for instance Plandool-G;
- copolymers of polyols and of diacid dimers, and esters thereof, such as Hailuscent ISDA, or the copolymer of dilinoleic acid/butanediol;
- fatty alcohols that are liquid at ambient temperature, with a branched and/or unsaturated carbon chain having 12 to 26 carbon atoms, such as 2-octyldodecanol, isostearyl alcohol, oleic alcohol, 2-hexyldecanol, 2-butyloctanol and 2-undecylpentadecanol;
- C₁₂-C₂₂ higher fatty acids, such as oleic acid, linoleic acid, linolenic acid, and mixtures thereof;
- dialkyl carbonates, the two alkyl chains may be identical or different, such as dicaprylyl carbonate marketed under the name CETIOL CC®, by COGNIS;
- oils of higher molar mass having in particular a molar mass ranging from approximately 400 to approximately 10,000 g/mol, in particular from approximately 650 to approximately 10,000 g/mol, in particular from approximately 750 to approximately 7,500 g/mol, and more particularly ranging from approximately 1,000 to approximately 5,000 g/mol. As oils of higher molar mass that can be used in the invention, mention may in particular be made of the oils selected from:
   - lipophilic polymers,
   - linear fatty acid esters having a total carbon number ranging from 35 to 70,
   - hydroxylated esters,
   - aromatic esters,
   - esters of C24-C28 branched fatty acids or fatty alcohols,
   - silicone oils,
   - oils of plant origin,
   - and mixtures thereof;
- fluorinated oils optionally partially hydrocarbon-based and/or silicone-based, such as fluorosilicone oils, fluorinated polyethers or fluorinated silicones, as described in document EP-A-847 752;
- silicone oils, such as polydimethylsiloxanes (PDMS) which are non-volatile and linear or cyclic; polydimethylsiloxanes comprising alkyl, alkoxy or phenyl groups which are pendant or at the end of the silicone chain, said groups having from 2 to 24 carbon atoms such as caprylyl methicone; phenylated silicones, such as phenyl trimethicones, phenyl dimethicones, phenyltrimethylsiloxydiphenylsiloxanes, diphenyl dimethicones, diphenylmethyldiphenyl-trisiloxanes or (2-phenylethyl)trimethylsiloxysilicates, and
- mixtures thereof.

Among the linear or branched hydrocarbons, of mineral or synthetic origin, paraffin oils or petroleum jelly are preferably used.

Among the hydrocarbon oils of plant origin, mention may be made, preferably, of plant oils, such as sweet almond oil, jojoba oil or macadamia nut oil.

### Additional dyes

The compositions according to the invention may also comprise a dye or a mixture of dyes, other than the pigments as defined above.

According to one embodiment, the additional dye content is 0.1% to 20%, preferably 0.5% to 15%, and more preferably 1% to 10%, by weight in relation to the total weight of said composition.

The additional dye (or coloring agent) present in the compositions according to the invention is chosen for example from the group consisting of colorants, nacres and reflective particles (or glitter).

A composition according to the invention may advantageously incorporate at least one dye selected from organic or inorganic dyes, in particular such as nacres conventionally used in cosmetic compositions, liposoluble or water-soluble coloring agents, materials with a specific optical effect, and mixtures thereof.

According to one embodiment, the additional dye is chosen from nacres.

The term "nacres" should be understood to mean iridescent or non-iridescent colored particles of any shape, which are in particular produced by certain mollusks in their shell or else are synthesized and which exhibit a color effect by optical interference.

The nacres may be present at a rate of 0.5% to 20%, preferably 1% to 15%, and more preferably 1% to 10%, by weight in relation to the total weight of said composition.

The nacres may be selected from pearlescent pigments such as bismuth oxychloride, titanium mica coated with iron oxide, titanium mica coated with bismuth oxychloride, titanium mica coated with chromium oxide, titanium mica coated with an organic dye, and pearlescent pigments based on bismuth oxychloride. This may also involve mica particles at the surface whereof are superposed at least two successive layers of metal oxides and/or of organic dyes.

By way of example of nacres, mention may also be made of natural mica coated with titanium oxide, with iron oxide, with natural pigment or with bismuth oxychloride.

Among the nacres available on the market, mention may be made of the TIMICA, FLAMENCO and DUOCHROME nacres (based on mica) marketed by ENGELHARD, the TIMIRON nacres marketed by MERCK, the nacres based on mica, PRESTIGE, marketed by ECKART and the nacres based on synthetic mica, SUNSHINE, marketed by SUN CHEMICAL.

The nacres may more particularly possess a yellow, pink, red, bronze, orange, brown, gold and/or copper color or glint.

By way of illustration of nacres which can be used in the context of the invention, mention may, in particular, be made of the gold nacres marketed, in particular, by ENGELHARD, under the name Brilliant gold 212G (Timica), Gold 222C (Cloisonne), Sparkle gold (Timica), Gold 4504 (Chromalite) and Monarch gold 233X (Cloisonne); the bronze nacres, marketed, in particular, by MERCK under the name Bronze fine (17384) (Colorona) and Bronze (17353) (Colorona) and by ENGELHARD under the name Super bronze (Cloisonne); the orange nacres, in particular, marketed by ENGELHARD under the name Orange 363C (Cloisonne) and Orange MCR 101 (Cosmica) and by MERCK under the name Passion orange (Colorona) and Matte orange (17449) (Microna); the brown-hued nacres marketed in particular by ENGELHARD under the name Nu-antique copper 340XB (Cloisonne) and Brown CL4509 (Chromalite); the copper-glint nacres marketed in particular by ENGELHARD under the name Copper 340A (Timica); the red-glint nacres marketed in particular by MERCK under the name Sienna fine (17386) (Colorona); the yellow-glint nacres marketed in particular by ENGELHARD under the name Yellow (4502) (Chromalite); the gold-glint red-hued nacres marketed in particular by ENGELHARD under the name Sunstone G012 (Gemtone); the pink nacres marketed in particular by ENGELHARD under the name Tan opal G005 (Gemtone); the gold-glint black nacres marketed in particular by ENGELHARD under the name Nu-antique bronze 240 AB (Timica), the blue nacres marketed in particular by MERCK under the name Matte blue (17433) (Microna), the silver-glint white nacres marketed in particular by MERCK under the name Xirona Silver and the green-gold and pinkish orangish nacres marketed in particular by MERCK under the name Indian summer (Xirona) and mixtures thereof.

According to one embodiment, the additional dye is chosen from colorants.

The term "colorants" refers to generally organic compounds soluble in fats such as oils or in a hydroalcoholic phase.

The composition according to the invention may also therefore comprise water-soluble or liposoluble coloring agents. The liposoluble colorants are, for example, Sudan Red, DC Red 17, DC Green 6, β-carotene, soybean oil, Sudan Brown, DC Yellow 11, DC Violet 2, DC Orange 5, and Quinoline Yellow. The water-soluble colorants are, for example, beetroot juice and caramel.

The compositions according to the invention may also contain at least one material with a specific optical effect, also referred to as glitter or reflective particles.

This effect is different from a simple conventional hue effect, i.e. a unified and stabilized effect of the kind produced by conventional dyes, such as, for example, monochromatic pigments. For the purpose of the invention, the term "stabilized" signifies absence of an effect of variability of color with the angle of observation or in response to a temperature change.

For example, this material may be selected from particles having a metallic glint, goniochromatic coloring agents, diffracting pigments, thermochromatic agents, optical brighteners, and also fibers, in particular of the interference type. Of course, these various materials may be combined so as to provide the simultaneous manifestation of two effects, or even a new effect in accordance with the invention.

The metallic-glint particles that can be used in the invention are in particular chosen from:
- particles of at least one metal and/or of at least one metal derivative,
- particles comprising a single-substance or multi-substance, organic or mineral substrate, at least partially coated with at least one metallic-glint layer comprising at least one metal and/or at least one metal derivative, and
- mixtures of said particles.

Among the metals that may be present in said particles, mention may, for example, be made of Ag, Au, Cu, Al, Ni, Sn, Mg, Cr, Mo, Ti, Zr, Pt, Va, Rb, W, Zn, Ge, Te, Se and mixtures or alloys thereof. Ag, Au, Cu, Al, Zn, Ni, Mo, Cr and mixtures or alloys thereof (for example, bronzes and brasses) are preferred metals.

The term "metal derivatives" denotes compounds derived from metals, in particular oxides, fluorides, chlorides and sulfides.

### Fillers

The compositions according to the invention may also comprise at least one filler, of organic or mineral nature, which makes it possible in particular to confer thereon additional properties of enhanced matteness, covering power, staying power and/or stability.

The term "filler" should be understood to mean colorless or white solid particles of any shape, which are in a form that is insoluble or dispersed in the medium of the composition. Mineral or organic in nature, they make it possible to confer body or rigidity on the composition, and/or softness, and uniformity on the makeup.

The fillers used in the compositions according to the present invention may be of lamellar, globular or spherical form, or in the form of fibers or in any other intermediate form between these defined forms.

The fillers may or may not be coated superficially, and in particular they may be surface-treated with silicones, amino acids, fluorinated derivatives or any other substance that promotes the dispersion and compatibility of the filler in the composition.

As examples of mineral fillers, mention may be made of talc, mica, silica, hollow silica microspheres, kaolin, calcium carbonate, magnesium carbonate, hydroxyapatite, boron nitride, glass or ceramic microcapsules, composites of silica and of titanium dioxide, such as the TSG series marketed by Nippon Sheet Glass.

As examples of organic fillers, mention may be made of polyamide powder (Nylon® Orgasol from Atochem), polyethylene powder, polymethyl methacrylate powder, polytetrafluoroethylene powder (Teflon), acrylic acid copolymer powder (Polytrap from Dow Corning), lauroyl lysine, hollow polymeric microspheres such as those of polyvinylidene/acrylonitrile chloride such as Expancel (Nobel Industries), hexamethylene diisocyanate/Trimethylol hexyllactone copolymer powder (Plastic Powder from Toshiki), silicone resin microbeads (Tospearl from Toshiba for example), synthetic or natural micronized waxes, metal soaps derived from organic carboxylic acids having 8 to 22 carbon atoms, and preferably from 12 to 18 carbon atoms, for example, zinc stearate, magnesium stearate or lithium stearate, zinc laurate, magnesium myristate, Polypore® L 200 (Chemdal Corporation), cross-linked elastomeric organopolysiloxane powders coated with silicone resin, in particular silsesquioxane resin, as described for example in the patent US 5 538 793, polyurethane powders, in particular, cross-linked polyurethane powders including a copolymer, said copolymer comprising trimethylol hexyllactone. In particular, it may be a polymer of hexamethylene diisocyanate/trimethylol hexyllactone. Such particles are in particular commercially available, for example under the trade name PLASTIC POWDER D-400® or PLASTIC POWDER D-800® from TOSHIKI, and mixtures thereof. This can also be cellulose powder such as that sold by Daito in the Cellulobeads range.

According to one particular embodiment of the invention, the composition comprises at least one cross-linked elastomeric organopolysiloxane powder.

The elastomeric organopolysiloxane powder(s) may be present at a content ranging from 0.5% to 12% by weight, advantageously from 1% to 8% by weight in relation to the total weight of said composition.

In particular, mention may be made of cross-linked elastomeric organopolysiloxane powders coated with silicone resin, in particular silsesquioxane resin, as described for example in the patent US 5 538 793. Such elastomer powders are sold under the trade names KSP-100®, KSP-101®, KSP-102®, KSP-103®, KSP-104® and KSP-105® by SHIN ETSU; mention may also be made of cross-linked elastomeric organopolysiloxane powders coated with silicone resin such as hybrid silicone powders functionalized by fluoroalkyl groups, in particular sold under the trade name "KSP-200" by Shin Etsu; or hybrid silicone powders functionalized by phenyl groups, in particular sold under the trade name "KSP-300" by Shin Etsu.

According to one advantageous embodiment, the compositions according to the invention comprise fillers chosen from silicone fillers, particularly KSP-100® and KSP-300®.

### Additives

A composition according to the invention may also further comprise any additive normally used in the field in question, for example chosen from gums, resins, dispersants, polymers, antioxidants, essential oils, preservatives, fragrances, neutralizing agents, antiseptic agents, anti-UV protective agents, cosmetic active agents, such as vitamins, hydrating agents, emollients or collagen-protecting agents, and mixtures thereof.

A person skilled in the art can adjust the type and amount of additives present in the compositions according to the invention by means of routine operations, so that the cosmetic properties and the stability properties sought for these compositions are not affected by the additives.

### Cosmetic compositions

The present invention also relates to a cosmetic composition including, in a physiologically acceptable medium, a composition such as defined above.

The term "physiologically acceptable medium" is intended to denote a medium that is particularly suitable for the application of a composition of the invention to the skin or the lips.

The physiologically acceptable medium is generally suitable for the nature of the support to which the composition should be applied, and also for the way in which the composition is to be packaged.

### Applications

The cosmetic compositions covered by the invention may be face or body treatment or makeup products.

The compositions according to the invention are cosmetic compositions intended for makeup and/or skincare.

Preferably, the compositions according to the invention are in the form of a foundation.

These compositions are thus intended to be applied onto the skin.

The present invention also relates to a non-therapeutic cosmetic skin treatment method comprising a step for applying at least one layer of a composition according to the invention onto the skin.

The present invention also relates to a non-therapeutic makeup and/or skincare method comprising a step for applying at least one layer of a composition as defined above onto the skin.

The present invention also relates to a skin makeup method wherein a composition as defined above is applied.

In particular, according to the invention, the composition can be applied with a finger or sponge.

Throughout the application, the term "comprising a" or "including a" means "comprising at least one" or "including at least one", unless otherwise specified.

Throughout the above description, unless specified otherwise, the term "between x and y" refers to an inclusive range, i.e. the values x and y are included in the range.

The invention will now be illustrated in the following non-limiting examples. Unless specified otherwise, the % are expressed by weight in relation to the total weight of the composition.

The compositions are prepared using routine cosmetic composition formulation methods.

### EXAMPLES

The various compositions were evaluated in respect of the appearance thereof, cosmetics thereof, particularly the application property thereof.

The compositions below are prepared and tested according to the protocols described hereinafter.

| | | Ex. 1 (invention) | Ex. 2 (invention) | Ex. 3 (invention) | Ex. 4 (comparative) |
|---|---|---|---|---|---|
| Phase | Chemical name | Content | Content | Content | Content |
| A1 | BIS-PEG/PPG-16/16 PEG/PPG-16/16 DIMETHICONE (and) CAPRYLIC/CAPRIC TRIGLYCERIDE (Abil Care 85, Evonik) | 0.8 | 0.8 | 0.8 | 0.8 |
| | ARACHIDYL ALCOHOL (and) BEHENYL ALCOHOL (and) ARACHIDYL GLUCOSIDE (Montanov 202, Seppic) | 1.9 | | | |
| | BEHENYL ALCOHOL (and) GLYCERYL STEARATE (and) DISODIUM ETHYLENE DICOCAMIDE PEG-15 DISULFATE (and) GLYCERYL STEARATE CITRATE (Ceralution H, Sasol) | | 1.9 | | |
| | BEHENYL ALCOHOL | | | 1.9 | |
| | Stearic acid | 0.45 | 0.45 | 0.45 | 0.45 |
| | Stearyl alcohol | 0.75 | 0.75 | 0.75 | 0.75 |
| | Cyclohexadimethicone | 9.7 | 9.7 | 9.7 | 9.7 |
| A2 | Pigments coated with Disodium Stearoyl Glutamate & Aluminum Hydroxide (=NAl from Myioshi) | 10 | 10 | 10 | 10 |
| | Cyclohexadimethicone | 5 | 5 | 5 | 5 |
| B | Water | Qsp100 | Qsp100 | Qsp100 | Qsp100 |
| | Glycerin | 5 | 5 | 5 | 5 |
| | Mono-di-palmito-stearate of sucrose (TEGO CARE SE 121 from EVONIK GOLDSCHMIDT) | 1.9 | 1.9 | 1.9 | 1.9 |
| C | Cyclohexadimethicone | 5 | 5 | 5 | 5 |
| | Steareth-100/PEG-136/HDI copolymer (RHEOLATE FX-1100, Elementis) | 3.5 | 3.5 | 3.5 | 3.5 |
| D | FILLER (KSP-300 Shin Etsu) | 1 | 1 | 1 | 1 |
| D | FILLER Cellulose | 1 | 1 | 1 | 1 |
| E | Preservatives | 0.5 | 0.5 | 0.5 | 0.5 |
| | Pentylene Glycol | 4 | 4 | 4 | 4 |

### Composition preparation protocol

The phase A1 is heated to a temperature that allows all of the constituents to be melted (about 75°-80°C) then the phase A2 is introduced into the phase A1 under Raynerie stirring, keeping the whole at 65°C.

The aqueous phase B is placed in Raynerie under stirring and heated to 70 so that all of the ingredients are melted, the temperature is then brought to 65°C. *Preparation of the emulsion*: still at 65°C, the phase A1+A2 is incorporated into B in the Raynerie and the whole is stirred for 10 minutes then the phase C is added (by increasing the speed if necessary). After 10 minutes the phase D is added and after 5 minutes the phase E is added. The composition is stirred again for 15 minutes.

### Evaluation of the compositions

The compositions of examples 1 and 2 comprise, as surfactants, mixtures of compounds comprising C20 and C22 fatty chains. For these compositions, the formulas obtained are compact and therefore have a satisfactory aspect while still allowing for a good application and a natural result.

The composition of example 3 comprises a C22 fatty alcohol. For this composition, the formula obtained is compact and therefore has a satisfactory aspect while still allowing for a good application and a natural result.

The composition of example 4 comprises a C18 surfactant and therefore does not contain any fatty acid or alcohol according to the invention. For this composition, the formula obtained is not compact but is rather soft and elastic.

It has therefore been shown that for a composition that does not contain fatty alcohol or acid according to the invention, namely an fatty alcohol or acid with a fatty chain comprising at least 20 carbon atoms, the formula obtained is not nomad as it is too liquid. In addition, taking the product on the finger or a sponge is not good because, as the product is too soft, the quantity of the product taken is excessive.

## Claims

1. Composition, in the form of an oil-in-water emulsion, containing:
- at least one associative polyurethane complying with the following formula (I): 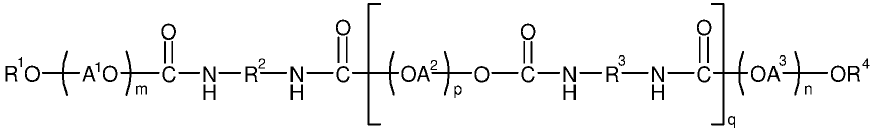 in which:
* R¹ and R⁴ represent, independently of each other, a linear or branched bivalent hydrocarbon radical, comprising 8 to 36 carbon atoms,
* A¹, A² and A³ represent, independently of each other, a linear or branched alkylene radical, having 2 to 4 carbon atoms;
* m and n represent, independently of each other, an integer between 0 and 1,000,
* p represents an integer between 5 and 500,
* q represents an integer between 1 and 8,
* R² and R³ represent, independently of each other, a linear or branched bivalent hydrocarbon radical, comprising 1 to 30 carbon atoms,
- at least one nonionic surfactant, said nonionic surfactant being chosen in the group consisting of: alkylpolyglucosides, gemini surfactants, silicone surfactants, and mixtures thereof;
- at least one fatty alcohol comprising a fatty chain containing at least 20 carbon atoms, and being chosen in the group consisting of: behenyl alcohol, arachidylic alcohol, and mixtures thereof;
- and at least one pigment chosen from among pigment particles coated by at least one lipophilic compound, wherein the coated pigment particles are iron oxide and/or titanium oxide particles coated with a lipophilic compound chosen from the group consisting of disodium stearoyl glutamate, isopropyl trisostearyl titanate, dimethicone, triethoxy caprylylsilane, hydrogenated lecithin and mixtures thereof,
wherein the content of associative polyurethane active substance having formula (I) is greater than 1.5% by weight in relation to the total weight of said composition.

2. Composition according to claim 1, comprising 2% to 10% by weight of associative polyurethane active substance having formula (I) in relation to the total weight of said composition.

3. Composition according to claim 1 or 2, wherein, in formula (I), A¹, A² and A³ represent an ethylene radical.

4. Composition according to any of claims 1 to 3, wherein, in formula (I), m and n represent, independently of each other, an integer between 1 and 500.

5. Composition according to any of claims 1 to 3, wherein, in formula (I), R¹ and R⁴ represent, independently of each other, a linear alkyl group comprising 8 to 36 carbon atoms.

6. Composition according to claim 4, wherein, in formula (I), R¹ and R⁴ represent a linear alkyl group comprising 18 carbon atoms.

7. Composition according to any of claims 1 to 6, wherein, in formula (I), m and n represent, independently of each other, an integer between 50 and 200.

8. Composition according to any of claims 1 to 7, comprising 0.1% to 10% by weight of fatty alcohol(s), in relation to the total weight of said composition

9. Composition according to any of claims 1 to 8, comprising 0.5% to 10% by weight of nonionic surfactant in relation to the total weight of said composition.

10. Composition according to any of claims 1 to 9, comprising at least one volatile oil, preferably at least one volatile silicone oil.

11. Composition according to any of claims 1 to 10, comprising at least one filler.

12. Composition, in particular cosmetic, according to any of claims 1 to 11, being a foundation.

13. Non-therapeutic skin makeup and/or skincare method, including a step for applying on the skin at least one layer of a composition according to claim 12.

## Patentansprüche

1. Zusammensetzung, in Form einer Öl-in-Wasser-Emulsion, enthaltend:
- mindestens ein assoziatives Polyurethan, das der folgenden Formel (I) entspricht: 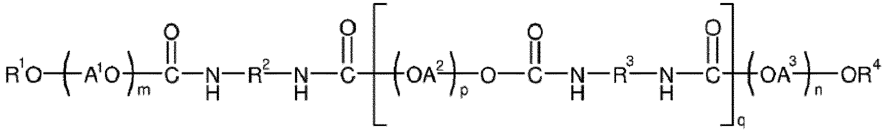 in der:
* R1 und R4, unabhängig voneinander, einen linearen oder verzweigten, zweiwertigen Kohlenwasserstoffrest darstellen, der 8 bis 36 Kohlenstoffatome aufweist,
* A1, A2 und A3, unabhängig voneinander, einen linearen oder verzweigten Alkylenrest darstellen, der 2 bis 4 Kohlenstoffatomen aufweist;
* m und n, unabhängig voneinander, eine ganze Zahl zwischen 0 und 1.000 darstellen,
* p eine ganze Zahl zwischen 5 und 500 darstellt,
* q eine ganze Zahl zwischen 1 und 8 darstellt,
* R2 und R3, unabhängig voneinander, einen linearen oder verzweigten, zweiwertigen Kohlenwasserstoffrest darstellen, der 1 bis 30 Kohlenstoffatome aufweist,
- mindestens ein nichtionisches Tensid, wobei das nichtionische Tensid ausgewählt ist aus der Gruppe bestehend aus: Alkylpolyglucosiden, Gemini-Tensiden, Silikon-Tensiden und deren Mischungen;
- mindestens einen Fettalkohol, der eine Fettkette mit mindestens 20 Kohlenstoffatomen aufweist und ausgewählt ist aus der Gruppe bestehend aus:
Behenylalkohol, Arachidylalkohol und deren Mischungen;
- und mindestens ein Pigment, ausgewählt aus Pigmentpartikeln, die mit mindestens einer lipophilen Verbindung beschichtet sind, wobei die beschichteten Pigmentpartikel Eisenoxid- und/oder Titanoxidpartikel sind, die mit einer lipophilen Verbindung, ausgewählt aus der Gruppe bestehend aus Dinatriumstearoylglutamat, Isopropyltrisostearyltitanat, Dimethicon, Triethoxycaprylylsilan, hydriertem Lecithin und deren Mischungen, beschichtet sind,
wobei der Gehalt an assoziativem Polyurethan-Wirkstoff mit der Formel (I) größer als 1,5 Gew.-% ist, bezogen auf das Gesamtgewicht der Zusammensetzung.

2. Zusammensetzung gemäß Anspruch 1, aufweisend 2 bis 10 Gew.-% des assoziativen Polyurethan-Wirkstoffs der Formel (I), bezogen auf das Gesamtgewicht der Zusammensetzung.

3. Zusammensetzung gemäß Anspruch 1 oder 2, wobei in Formel (I) A1, A2 und A3 einen Ethylenrest darstellen.

4. Zusammensetzung gemäß irgendeinem der Ansprüche 1 bis 3, wobei in Formel (I) m und n, unabhängig voneinander, eine ganze Zahl zwischen 1 und 500 darstellen.

5. Zusammensetzung gemäß irgendeinem der Ansprüche 1 bis 3, wobei in Formel (I) R1 und R4, unabhängig voneinander, eine lineare Alkylgruppe mit 8 bis 36 Kohlenstoffatomen darstellen.

6. Zusammensetzung gemäß Anspruch 4, wobei in Formel (I) R1 und R4 eine lineare Alkylgruppe mit 18 Kohlenstoffatomen darstellen.

7. Zusammensetzung gemäß irgendeinem der Ansprüche 1 bis 6, wobei in Formel (I) m und n, unabhängig voneinander, eine ganze Zahl zwischen 50 und 200 darstellen.

8. Zusammensetzung gemäß irgendeinem der Ansprüche 1 bis 7, aufweisend 0,1 bis 10 Gew.-% Fettalkohol(e), bezogen auf das Gesamtgewicht der Zusammensetzung.

9. Zusammensetzung gemäß irgendeinem der Ansprüche 1 bis 8, aufweisend 0,5 bis 10 Gew.-% nichtionisches Tensid, bezogen auf das Gesamtgewicht der Zusammensetzung.

10. Zusammensetzung gemäß irgendeinem der Ansprüche 1 bis 9, aufweisend mindestens ein flüchtiges Öl, vorzugsweise mindestens ein flüchtiges Silikonöl.

11. Zusammensetzung gemäß irgendeinem der Ansprüche 1 bis 10, aufweisend mindestens einen Füllstoff.

12. Zusammensetzung, insbesondere eine kosmetische, gemäß irgendeinem der Ansprüche 1 bis 11, die eine Grundierung darstellt.

13. Nicht-therapeutische Hautschminke und/oder Hautpflegeverfahren, aufweisend einen Schritt des Auftragens von mindestens einer Schicht einer Zusammensetzung gemäß Anspruch 12 auf die Haut.

## Revendications

1. Composition, sous la forme d'une émulsion huile-dans-eau, contenant :
- au moins un polyuréthane associatif répondant à la formule (I) suivante : 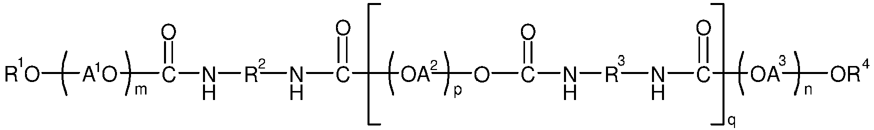 dans laquelle :
* R¹ et R⁴ représentent, indépendamment l'un de l'autre, un radical hydrocarboné linéaire ou ramifié comprenant de 8 à 36 atomes de carbone,
* A¹, A² et A³ représentent, indépendamment l'un de l'autre, un radical alkylène, linéaire ou ramifié, ayant de 2 à 4 atomes de carbone ;
* m et n représentent, indépendamment l'un de l'autre, un nombre entier compris entre 0 et 1 000,
* p représente un nombre entier compris entre 5 et 500,
* q représente un nombre compris entre 1 et 8,
* R² et R³ représentent, indépendamment l'un de l'autre, un radical hydrocarboné bivalent, linéaire ou ramifié, comprenant de 1 à 30 atomes de carbone,
- au moins un tensioactif non ionique, ledit tensioactif non ionique étant choisi dans le groupe constitué des alkylpolyglycosides, des tensioactifs géminés, des tensioactifs siliconés, et de leurs mélanges
- au moins un alcool gras contenant une chaîne grasse comprenant au moins 20 atomes de carbone, et étant choisi dans le groupe constitué de l'alcool béhénique, de l'alcool arachidylique et de leurs mélanges,
- et au moins un pigment, choisi parmi les particules de pigment enrobé par au moins un composé lipophile, les particules de pigment enrobé étant des particules d'oxydes de fer et/ou d'oxyde de titane enrobées par un composé lipophile choisi dans le groupe constitué du disodium stéaroyl glutamate, du trisostéaryle titanate d'isopropyle, de la diméthicone, du triéthoxy caprylylsilane, de la lécithine hydrogénée et de leurs mélanges,
dans laquelle la teneur de matière active de polyuréthane associatif de formule (I) est strictement supérieure à 1,5% en poids par rapport au poids total de ladite composition.

2. Composition selon la revendication 1, comprenant de 2% à 10% en poids de matière active de polyuréthane associatif de formule (I) par rapport au poids total de ladite composition.

3. Composition selon la revendication 1 ou 2, dans laquelle, dans la formule (I), A¹, A² et A³ représentent un radical éthylène.

4. Composition selon l'une quelconque des revendications 1 à 3, dans laquelle, dans la formule (I), m et n représentent, indépendamment l'un de l'autre, un nombre entier compris entre 1 et 500.

5. Composition selon l'une quelconque des revendications 1 à 4, dans laquelle, dans la formule (I), R¹ et R⁴ représentent, indépendamment l'un de l'autre, un groupe alkyle linéaire comprenant de 8 à 36 atomes de carbone.

6. Composition selon la revendication 5, dans laquelle, dans la formule (I), R¹ et R⁴ représentent un groupe alkyle linéaire comprenant 18 atomes de carbone.

7. Composition selon l'une quelconque des revendications 1 à 6, dans laquelle, dans la formule (I), m et n représentent, indépendamment l'un de l'autre, un nombre entier compris entre 50 et 200.

8. Composition selon l'une quelconque des revendications 1 à 7, comprenant de 0,1% à 10% en poids d'acide(s) gras ou d'alcool(s) gras, par rapport au poids de ladite composition

9. Composition selon l'une quelconque des revendications 1 à 8, comprenant de 0,5% à 10% en poids de tensioactif non ionique par rapport au poids total de ladite composition.

10. Composition selon l'une quelconque des revendications 1 à 9, comprenant au moins une huile volatile, de préférence siliconée.

11. Composition selon l'une quelconque des revendications 1 à 10, comprenant au moins une charge.

12. Composition, notamment cosmétique, selon l'une quelconque des revendications 1 à 11, sous la forme d'un fond de teint.

13. Procédé non thérapeutique de maquillage et/ou de soin de la peau comprenant une étape d'application sur la peau d'au moins une couche d'une composition selon la revendication 12.
